# EUROPEAN PATENT SPECIFICATION

(11) **EP 0 373 638 B1**
(45) Date of publication and mention of the grant of the patent: **13.10.1993**
(21) Application number: 89123073.2
(22) Date of filing: 13.12.1989
(51) Int. Cl.: B63C 11/18

(54) **Offset anatomical mouthpiece**
Abgestuftes anatomisches Mundstück
Embouchure anatomique échelonnée

(30) Priority: 13.12.1988 US 283611
(43) Date of publication of application: 20.06.1990
(73) Proprietor: Kinkade, Donald James, Greenley Colorado (US)
(72) Inventor: Kinkade, Donald James, Greenley Colorado (US)
(74) Representative: Feldkamp, Rainer, Dipl.-Ing.

(56) References cited:
- GB-A- 325 236
- GB-A- 1 173 293
- US-A- 3 415 245
- US-A- 3 768 465
- US-A- 4 466 434

## Description

The present application relates to mouthpieces which are used in underwater breathing devices such as snorkels and regulators and to mouthpieces which are used in inhalators and gastric tubes for medical treatment.

Such an asymmetrical mouthpiece is shown in US-A-4 466 434 and comprises an offset or lip between the upper and lower portions. Bite lugs or internal wing members on the legs of the U shape are shown which are of constant thickness and width and which are connected to each other by a ledge which forms the offset between the upper and lower portion to form a place for the upper front teeth to rest.

In spite of the fact that this mouthpiece constitutes an improvement over other known mouthpieces as shown f.i. in US-A-3 415 245 which are symmetrical on their upper and lower surfaces with bite lugs which are of constant thickness and width, the mouthpiece of US-A-4 466 434 is not adaptable for both overbite and underbite conditions. The US-A-4 466 434 mouthpiece cannot be used with comfort in an underbite condition because of the ledge formed between the bite lugs.

The object of the present invention is to provide a mouthpiece giving more people more comfort while using it and to increase safety due to a better fit of the mouthpiece.

This object is obtained by the features set out in claim 1.

Advantageous developments of the mouthpiece are set out in the subclaims.

The present invention provides a mouthpiece with an offset between upper and lower jaws combined with tapered bite lugs and a particular bite plane which can be used by persons who require a mouthpiece such as divers and medical patients which mouthpiece does not distort the normal rest position of the jaw bones and jaw muscles including but not limited to the ball joint assembly in the jaw. The present invention provides a mouthpiecewhich can be used with comfort by persons who have either an overbite or an underbite condition.

The invention is described in detail in the following with reference to embodiments shown in the drawings, wherein:
- Fig. 1: shows a top view of one embodiment of the mouthpiece.
- Fig. 2: shows a front view of the embodiment shown in Fig. 1.
- Fig. 3: is a rear view of the embodiment shown in Fig. 1.
- Fig. 4: is a view partially in section taken along the lines A-A shown in Fig. 2.
- Fig. 5 and 6: are respectively top and rear views of a second embodiment of the mouthpiece.
- Fig. 7 and 8: are top and front views of a third embodiment of the mouthpiece.

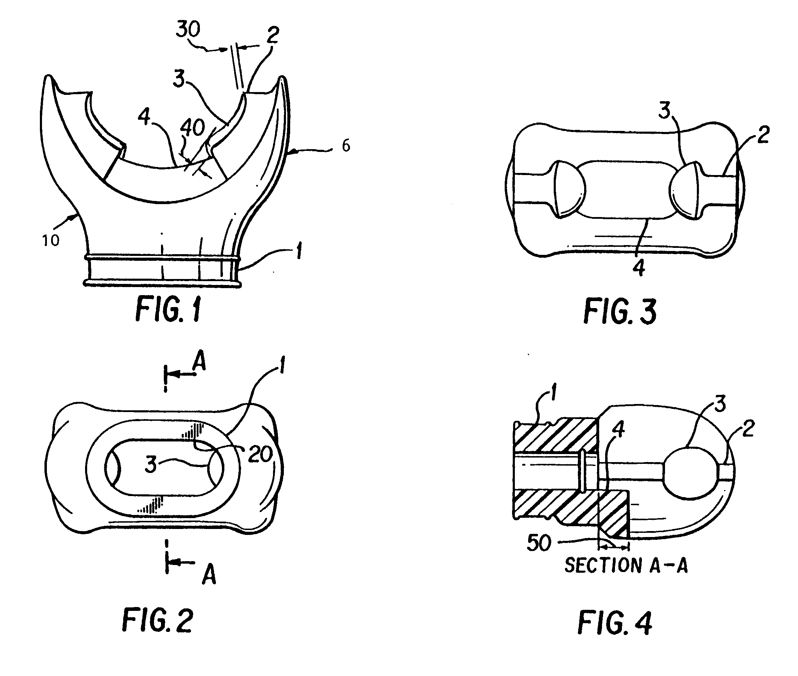

Fig. 1 shows a top view of one embodiment 10 of the mouthpiece according to the present invention formed of a moldable resilient material in which a standard coupling 1 is formed on one end. As shown in Fig. 2, the coupling 1 has an orifice 20 to permit the passage of a gas. On the other end of the U shaped body 6 which is formed into two legs, internal wings 3 are formed in either side of the generally U shaped body 6 on the inside thereof. A biteplane is designated with 2. The wings 3 have a taper as shown by the different thickness dimensions 30 and 40 which are different by about 2 (1-4) mm. The internal wing members 3 range from 6 to 12 mm in width, 14 to 40 mm in length and 2 to 8 mm in thickness and may be formed of a material which will conform to the shape of the user's teeth.

Fig. 4 shows a side view partially in section in which the offset nature of the mouthpiece is illustrated. A offset or lip 4 is formed on what is shown in this drawing as the lower portion of the U shaped body 6. The width 50 of the lip ranges from about 1 to about 14 mm depending on the amount of overbite or underbite the user has. Because the lip only slightly exeeds the extent of the orifice 20 and does reach to the internal wings 3, the inventive device can be used by persons with either an overbite or underbite. The lip 4 extends to the lower level of the wing 3 in a mouthpiece oriented for use with an overbite. Naturally, if the mouthpiece were oriented for use with an underbite, the lip 4 would extend to the upper level of the wing 3.

This further could simply be obtained by turning over the mouthpiece as shown in Fig. 4 by 180 degrees about the horizontal median line of this Figure.

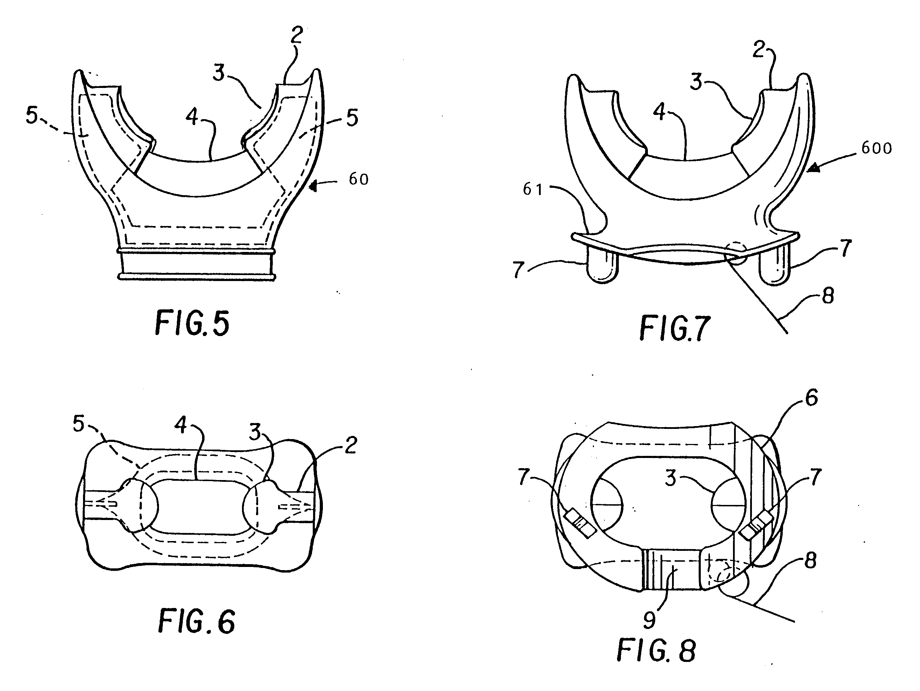

Fig. 5 and 6 show top and front views of a second embodiment of the present invention in which a stiffener 5 formed in the same general U shape is added to the body member 60 of the mouthpiece. The stiffener 5 may be formed of a rigid or slightly resilient material.

Fig. 7 and 8 show top and front views of a third embodiment of the present invention. This embodiment is substantially identical with the first embodiment shown in Fig. 1 through 4 except that clips 7 are attached to the mouthpiece 10 as shown in Fig. 7 and 8. A line or lanyard 8 may be attached to the clips. The outer edge of the U shaped structure has a rolled edge 61 formed thereon as shown in Fig. 7 and 8 to provide a convenient gripping surface for the clips 7. A channel or slot 9 may be formed in either the upper or the lower surface of the U shaped body member 600 to permit its removal without disconnecting any tubes which may be fitted for the user. This mouthpiece further protects such tubes from being pinched off by the teeth of the user.

The present invention has been described with reference to particular embodiments. However those skilled in the art understand that many changes and modifications of those embodiments are possible which are within the scope of the present invention.

## Claims

1. A mouthpiece comprising:
an U shaped body member (6;60;600) having first and second ends and upper and lower portions;
the first end forming a coupling (1) of generally oval shape in cross section and having an orifice (20) formed therein;
the second end extending away from the orifice (20) and forming the two leg members of said U shape, each leg member having a free end;
an internal wing member (3) formed on the inner side of each leg member near the free end thereof;
the lower portion of the U shaped body member (6;60;600) extending outwardly toward the leg members more than the upper portion of the U shaped body member (6;60;600) to form an offset (4);
characterized in that:
the internal wing members (3) have a taper such that, in a direction from the orifice (20) to the free ends of the leg members, first ends farther from the orifice (20) have a thickness (30) smaller than the thickness (40) of second ends nearer the orifice;

2. A mouthpiece as claimed in claim 1 wherein the offset (4) extends up to a level below the internal wing members (3).

3. A mouthpiece as claimed in claim 1 or 2, wherein the width (50) of the offset (4) ranges from 1 to 14 mm.

4. A mouthpiece as claimed in any of the claims 1 through 3, wherein the offset (4) only slightly exeeds the extent of the orifice (20) and does not reach the internal wing members (3).

5. A mouthpiece as claimed in any of the claims 1 through 4, wherein the internal wing members (3) are formed of material adapted to conform to the bite of the user.

6. A mouthpiece as claimed in any of the claims 1 through 5, including further an internal stiffener (5) formed in the U shaped body member (60).

7. A mouthpiece as claimed in any of the claims 1 through 6, wherein:
the U shaped body member (600) has an outer edge formed into a rolled edge (61);
clip members (7) attached to the rolled edge (61).

8. A mouthpiece claimed in any of the claims 1 through 7, wherein a channel (9) is formed through the U shaped member upper or lower portion for permitting the removal of tubes without disconnection.

9. A mouthpiece as claimed in any of the claims 1 through 8, wherein the internal wing members (3) have a range in size from 6 to 12 mm in width, from 14 to 40 mm in length and from 2 to 8 mm in thickness.

## Patentansprüche

1. Ein Mundstück mit:
einem U-förmigen Körperteil (6;60;600) mit ersten und zweiten Enden und oberen und unteren Abschnitten;
wobei das erste Ende eine Kupplung (1) mit allgemein ovaler Form im Querschnitt bildet, die eine darin ausgebildete Öffnung (20) aufweist;
wobei sich das zweite Ende von der Öffnung (20) fort erstreckt und die beiden schenkelteile der U-form bildet, wobei jedes Schenkelteil ein freies Ende aufweist;
einem inneren Flügelteil (3), das auf der Innenseite jedes Schenkelteils in der Nähe von dessen freiem Ende ausgebildet ist;
wobei sich der untere Abschnitt des U-förmigen Körperteils (6;60;600) weiter nach außen in Richtung auf die Schenkelteile erstreckt, als der obere Abschnitt des U-förmigen Körperteils (6;60;600), um eine Versetzung (4) zu bilden; dadurch gekennzeichnet, daß:
die inneren Flügelteile (3) eine derartige Verjüngung aufweisen, daß in einer Richtung von der Öffnung (20) zu den freien Enden der Schenkelteile erste von der Öffnung (20) weiter entfernte Enden der Flügelteile eine Dicke (30) aufweisen, die kleiner als die Dicke (40) von zweiten Enden näher an der Öffnung ist.

2. Mundstück nach Anspruch 1, bei dem sich die Versetzung (4) bis zu einer Höhenlage unterhalb der innenliegenden Flügelteile (3) erstreckt.

3. Mundstück nach Anspruch 1 oder 2, bei dem die Breite (50) der Versetzung (4) im Bereich von 1 bis 14 mm liegt.

4. Mundstück nach einem der Ansprüche 1 bis 3, bei dem die Versetzung (4) lediglich geringfügig die Erstreckung der Öffnung (20) überschreitet und nicht bis zu den innenliegenden Flügelteile (3) reicht.

5. Mundstück nach einem der Ansprüche 1 bis 4, bei dem die innenliegenden Flügelteile (3) aus einem Material gebildet ist, das sich an den Biß des Benutzers anpassen kann.

6. Mundstück nach einem der Ansprüche 1 bis 5, das ein inneres Versteifungsteil (5) einschließt, das in dem U-förmigen Körperteil (60) ausgebildet ist.

7. Mundstück nach einem der Ansprüche 1 bis 6, bei dem:
das U-förmige Körperteil (600) eine zu einer vorspringenden Kante (61) ausgebildete Außenkante aufweist;
wobei Klammerteile (7) an der vorspringenden Kante (61) befestigt sind.

8. Mundstück nach einem der Ansprüche 1 bis 7, bei dem ein Kanal (9) durch den oberen oder unteren Abschnitt des U-förmigen Körperteils hindurch ausgebildet ist, um die Entfernung von Schläuchen ohne Trennung zu ermöglichen.

9. Mundstück nach einem der Ansprüche 1 bis 8, bei dem die innenliegenden Flügelteile (3) eine Breite im Bereich von 6 bis 12 mm, eine Länge im Bereich von 14 bis 40 mm und eine Dicke im Bereich von 2 bis 8 mm aufweisen.

## Revendications

1. Embouchure comprenant :
un corps (6 ; 60 ; 600) en forme de U comportant des première et seconde extrémités et des parties supérieure et inférieure ;
la première extrémité formant un élément d'accouplement (1) de forme de section droite ovale dans son ensemble et comportant un orifice (20) qui y est formé ;
la seconde extrémité s'étendant dans une direction opposée à l'orifice (20) et formant les deux branches de ladite forme en U, chaque branche comportant une extrémité libre ;
une aile intérieure (3) formée sur le côté intérieur de chaque branche près de l'extrémité libre de cette dernière.
la partie inférieure du corps (6 ; 60 ; 600) en forme de U s'étendant vers l'extérieur en direction des branches sur une distance plus grande que la partie supérieure du corps (6 ; 60 ; 600) en forme de U de manière à former un rebord ou saillie (4);
caractérisée en ce que :
les ailes intérieures (3) présentent une variation progressive d'épaisseur ou effilement tel que, dans une direction allant de l'orifice (20) vers les extrémités libres des branches, les premières extrémités plus près de l'orifice (20) ayant une épaisseur (30) plus petite que l'épaisseur (40) des secondes extrémités plus près de l'orifice.

2. Embouchure selon la revendication 1, dans laquelle le rebord ou saillie (4) s'étend jusqu'à un niveau situé en dessous des ailes intérieures (3).

3. Embouchure selon la revendication 1 ou 2, dans laquelle la largeur (50) du rebord ou saillie (4) est comprise entre 1 et 14 mm.

4. Embouchure selon l'une quelconque des revendications 1 à 3, dans laquelle la saillie (4) ne dépasse que légèrement l'étendue de l'orifice (20) et n'atteint pas les ailes intérieures (3).

5. Embouchure selon l'une quelconque des revendications 1 à 4, dans laquelle les ailes intérieures (3) sont formées d'une matière adaptée pour se conformer au serrage par les dents de l'utilisateur.

6. Embouchure selon l'une quelconque des revendications 1 à 5, comprenant en outre un élément de renforcement intérieur (5) formé dans le corps (60) en forme de U.

7. Embouchure selon l'une quelconque des revendications 1 à 6, dans laquelle :
le corps (600) en forme de U comporte un bord extérieur se présentant sous la forme d'un bord roulé (61);
des pinces (7) sont fixées au bord roulé (61).

8. Embouchure selon l'une quelconque des revendications 1 à 7, dans laquelle une rainure (9) est formée à travers la partie supérieure ou la partie inférieure du corps en forme de U pour permettre l'enlèvement des tubes sans déconnexion.

9. Embouchure selon l'une quelconque des revendications 1 à 8, dans laquelle les ailes intérieures (3) ont une largeur comprise entre 6 et 12 mm, une longueur comprise entre 14 et 40 mm et une épaisseur comprise entre 2 et 8 mm.
